# EUROPEAN PATENT SPECIFICATION

(11) **EP 1 634 060 B1**
(45) Date of publication and mention of the grant of the patent: **25.12.2013**
(21) Application number: 04736476.5
(22) Date of filing: 09.06.2004
(51) Int. Cl.: G01N 21/31, G01N 21/64, G01N 21/65

(54) **Method and device for analysis, verification and quality assurance of drugs for infusion**
Verfahren und Vorrichtung zur Analyse, Verifizierung und Qualitätssicherung von Arzneistoffen zur Infusion
Procédé et dispositif permettant d'analyser, de vérifier et de contrôler la qualité de médicaments destinés à être infusés

(30) Priority: 10.06.2003 SE 0301663
(43) Date of publication of application: 15.03.2006
(73) Proprietor: Pharmacolog i Uppsala AB, 752 75 Uppsala (SE)
(72) Inventor: LENNERNÄS, Bo, S-451 44 Uddevalla (SE)
(74) Representative: Brann AB
(86) International application number: PCT/SE2004/000900
(87) International publication number: WO 2004/109262

(56) References cited:
- WO-A2-02/12853
- DE-A1- 19 713 249
- US-A- 5 750 998
- US-A- 5 956 144
- US-A- 6 111 639
- US-B1- 6 188 474
- US-B1- 6 410 255
- DATABASE WPI Week 198304, Derwent Publications Ltd., London, GB; Class B04, AN 1983-09145K, XP002994647 & SU 913 255 B (GEOC-R)GEOCHEM ANALYT CHEM(PHAR-R) PHARMACEVITIC RES IN) 15 March 1982

## Description

### Field of the invention

The present invention relates to a method and device for non-invasive analysis of the identity and concentration of drugs that are to be administered by infusion.

### Background of the invention

Administration by injection or infusion of drug solutions is performed under medically controlled conditions. The injection or infusion rate and other technical parameters are monitored by the medical personnel and the well-being of the patient is also monitored by regular observations. Records of the diagnosis of the patient's disease and planned treatment thereof are usually stored in the hospital databases where the hospital pharmacy can find the name of the prescribed drug as well as the prescription of the drug specified for each individual patient. The preparation of the drug is ordered by the treating physician for each patient at the time of administration. The drug preparation process is usually performed at the hospital pharmacy. The pharmacy personnel receive the prescription through the hospital database and mix the prescribed drug solution in accordance therewith. The risk for mistakes by the personnel can never be totally avoided since the mixture is made manually. Another weak link in the administration procedure regarding the quality assurance and the safety of the patient is when the prepared drug is transferred from the hospital pharmacy to the location where the administration will take place since many different drug preparations are handled at the same time and by different hospital personnel. There is an obvious risk for mix-up of different medicaments at the moment when the drug container is connected to the device used for administration. In many instances the final preparation of the drug is performed locally at the hospital ward by nurses that have limited pharmaceutical training. Since drugs for injection or infusion often are highly potent, errors in drug composition and concentration have very serious effects on the patient and can even be lethal.

Miscomprehension in drug prescriptions, which can involve poor handwriting, confusion between drugs with similar names, misuse of zeroes and decimal points, confusion of metric and other dosing units, and inappropriate abbreviations is known as one of the most common errors in medication in hospitals.

In the field of analytical methods for chemical solutions different types of spectrophotometric methods and other non-invasive test systems have been used over the years. A wide range of analytical equipment is available on the market providing robust and cost-effective analyses. Methods such as absorption spectrophotometry in the infrared, visible or ultraviolet wavelength range, fluorescence spectrophotometry, Raman spectrophotometry, nuclear magnetic resonance (NMR) as well as electron spin resonance (ESR) are widely used for this purpose. These methods can differentiate between different molecules in a solution by identifying a unique response profile for each kind of molecule and the concentration can be determined by the magnitude of the response. The result of these analyzing techniques provides both qualitative and quantitative information of the analysed solution.

As earlier described, there is the risk for administering an incorrect concentration of a prescribed drug or a different drug than the one prescribed, which often has very serious consequences for the patient. A final check and verification of the content in the drug container, by analyzing whether it contains the correct drug or not, and if the concentration of it is as prescribed, would eliminate the severe consequences of any mistake. Systems for performing such a check and verification have been proposed previously.

US 5,750,998 discloses an apparatus for non-invasively identifying components of a liquid medium within a flexible transparent bag. The apparatus comprises spacer means for supporting the bag, a source of electromagnetic radiation for directing electromagnetic radiation into the bag, detector means located outside the bag for receiving electromagnetic radiation which has passed through the bag, and means for analyzing the signal to identify the components of the liquid medium of the bag.

US 6,111,639 discloses a method and apparatus for testing the dosage and concentration of a medication in a flexible plastic bag. Prior to infusion with medication sealed into the bag, a suitable coloring agent, coded to provide identifying data relative to a particular medication, is added to the contents of the bag. The bag is then subjected to a spectrophotographic analysis by which the concentration and dosage of the medication in the bag is determined. The analysis is performed in an analyzer comprising a slot through which the intravenous bag is inserted, and a light source and a photocell arranged on opposite sides of the slot. The results of the analysis are compared with preset parameters for the dosage and concentration of the medication to provide an indication of the dosage and concentration.

### Summary of the invention

According to the present invention an improved method and device for checking and verifying the content of in a drug container has been developed.

In a first aspect, the invention provides a method to perform verification and quality assurance of a drug solution to be administered by infusion as defined in claim 1.

A characterizing feature of the method is that the drug container is coupled via a sterile tubing to a sterile analysing container, and that for spectrophotometric determination of the drug in the drug solution the analyzing container is placed in an analysing unit, drug solution is flowed through the sterile tubing to the sterile analyzing container, and one or more drug property values are determined by absorption spectrophotometry or fluorescence spectrometry or a combination thereof.

In a preferred embodiment of the method absorption spectrophotometry is used for determination of the drug solution content.

In another embodiment the method comprises determination of the identity of a drug solution.

In another embodiment the method comprises determination of the concentration of the drug solution.

In a further embodiment of the method a warning message is issued to stop the procedure if an agreement is not reached between the obtained profile and the profile of the prescribed drug.

In a second aspect of the invention there is provided a device for verification of correctness and quality assurance of a drug solution to be administered to a patient by infusion as defined in claim 10.

In an optional embodiment an external information network comprising the stored set of known profiles in an external database to which the obtained profile is compared, coupled to the central computation unit.

In a second optional embodiment the central computation unit is coupled to a patient treatment recording system.

In a third optional embodiment the central computation unit is coupled to a treatment planning system.

### Brief description of the drawings

Figure 1. Illustrates one embodiment of a system according to the invention.
Figure 2. Examples of spectral profiles for different drugs for infusion or injection.
Figure 3. Illustrates an analytical unit using light detection.

### Detailed description of the invention

For the purpose of this application the term "profile" is taken to mean as the spectrum drawn up in a graph as the result of an analytical test run for a certain drug.
The term "data base" comprises for the purpose of this application Electronic Patient Record Systems and Chemotherapy Management Systems.

The inventors have surprisingly found that it is possible to use a non-invasive determination of the chemical profile of a drug for quantitative and qualitative quality assurance and verification in connection with administration of drugs in liquid form.
The method according to the invention is based on a non-invasive analysis of the drug solution by spectrophotometric methods such as absorption spectrophotometry in the infrared, visible or ultraviolet wavelength range or fluorescence spectrophotometry.
These methods can differentiate between different molecules in a solution by identifying a unique response profile for each kind of molecule. The concentration of the component(s) is/are determined by measurement of the magnitude of the response to be compared with the magnitude of response of known concentrations for the substance(s) of interest. A set of known profiles comprising information on chemical identity and concentrations for each profile is recorded and stored in a database. These profiles are used as a reference for comparison against the results from a non-invasive analysis of a drug to be administered. A final testing of identity and concentration of the drug to be administered increases the safety for both the patients as well as for the medical personnel. This quality control should be performed at the preparatory stage of the treatment procedure and before the actual administration of the drug to the patient.
The data base unit is connected to an analytical unit such as an absorption spectrophotometer, and/or fluorescence spectrophotometer, that has its detection means coupled to the drug delivery system. The analytical unit determines the chemical profile of the content in the drug container and compares it to the profiles stored in the data base. When the determined chemical profile is found among the profiles in the database the concentration of the drug is calculated from the magnitude of the response. The result of the analysis is displayed on the analysing unit, printed as a treatment record and/or transferred to a patient database where all treatment parameters for each patient are stored. If the prescription is available from the hospital databases it can be compared with the result from the analysis and, if the data agree with regard to both type of drug and concentration, the staff is informed that it is safe to deliver this drug to the patient.

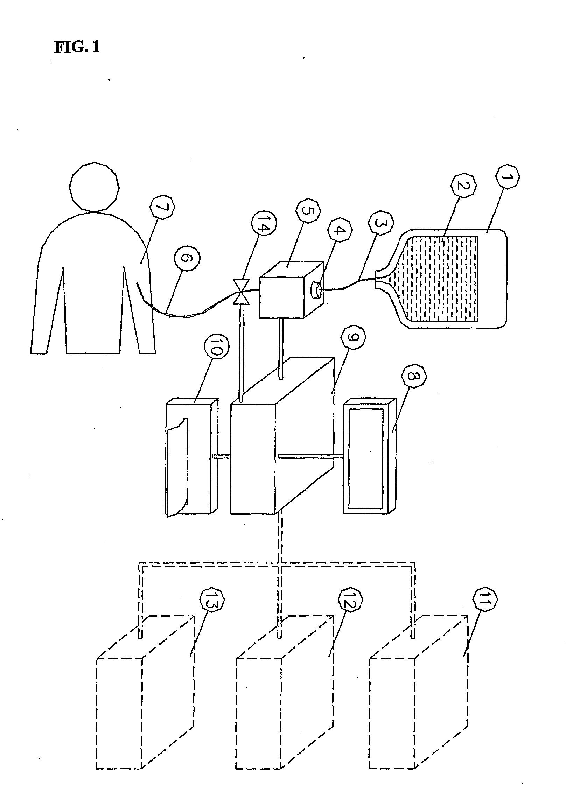

In one preferred embodiment of this invention, as shown in Figure 1, a drug container (1) contains a sterile drug solution (2) for infusion into a patient. The drug solution flows through a sterile tubing (3) to a sterile analyzing container (4) that is located in the analyzing unit (5). The analyzing unit comprises optical components for absorption spectrum analysis and/or fluorescence spectrum analysis. The signal from the analyzing unit (5) is transmitted to a central computation unit (9) where the measured spectrum, also referred to as profile, is compared to known profiles in a local database. If agreement is found with a previously stored profile for a drug solution the result is presented on a display unit (8) or printed on a printer (10). This system can also be coupled to an external information network where the reference profiles are taken from an optional external database (11) and the result of the analysis is transmitted to a patient treatment management system (12) for recording. The external information network can also supply data about the prescribed treatment for the particular patient from a treatment planning system (13) and if the controlled identity and concentration of the drug solution agrees with the prescription for this patient the system issues a message that the treatment is safe and the infusion process can start. The system may also include a control valve (14) that opens for infusion through the tubing (6) and needle (7) only if the measured profile agrees with the reference profile for the prescribed drug solution for this particular patient.

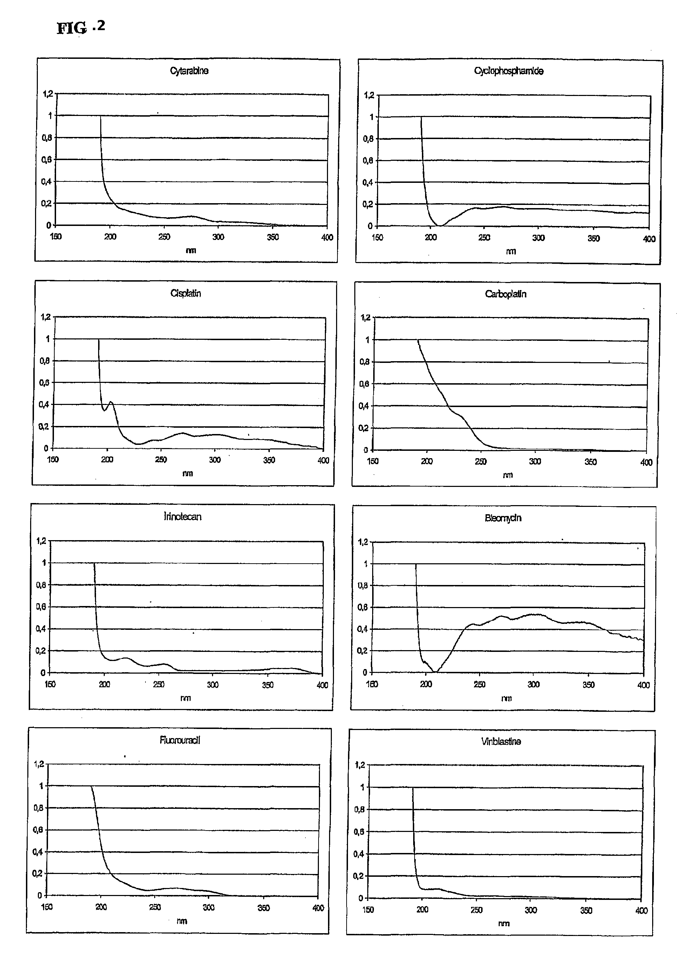

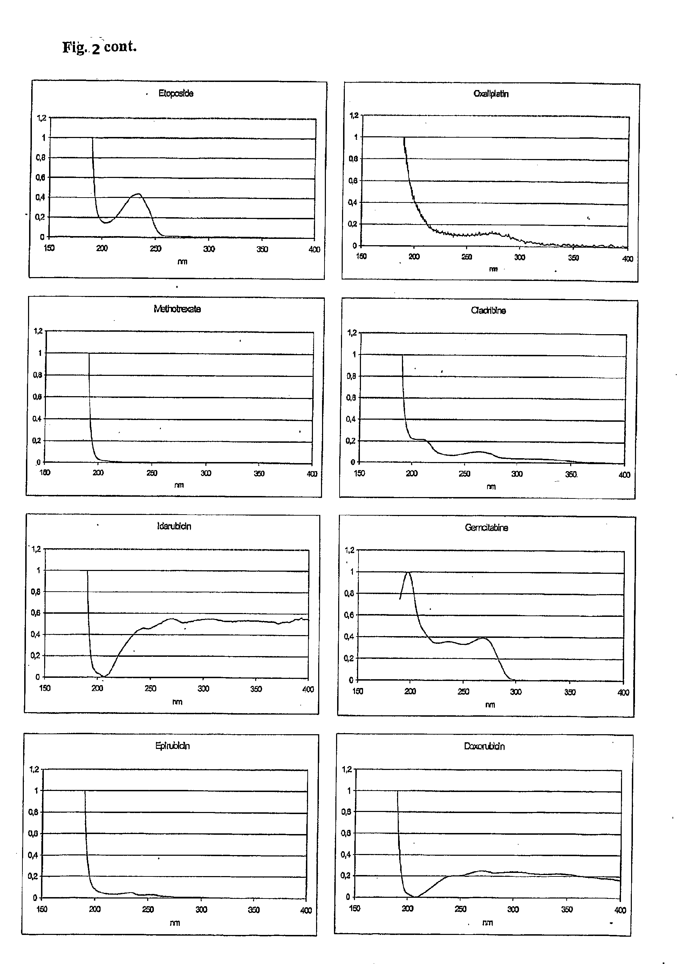

The aim of the following description is to show the unique individual characteristics of drug profiles obtained by absorption spectrophotometry. Figure 2 shows the absorption spectra in the ultraviolet wavelength range 190 - 400 nm for a number of commonly used chemotherapy drugs. The illustrated spectra are normalized to the maximum absorption level in order to show the differences in the profiles for the different drug solutions. The profiles show that these drugs have unique profiles that can be clearly separated from each other to identify the type of drug with a high degree of certainty. For applications where the UV spectral profiles of the used drug solutions are too similar to give certain identification, the wavelength range can be extended to the visible and infrared range or complementary techniques such as fluorescence spectrophotometry can be used.

One important application for this invention is the verification of the drug solution used for chemotherapy against cancer. Here the prescription is tailored for each individual patient and any errors in the type of drug and/or concentration has very serious effects that even can be lethal.
Other potential applications for this invention are intensive care, where nutrition, pain relief and/or medication is given by infusion, dialysis, anaesthesia, surgery, where blood transfusion, blood plasma infusion or blood recovery is used, or any other procedure where drugs or other substances are given to a patient through infusion.

The objective of the following description is to further characterise the analyzing unit of the invention. The analyzing unit (5) in figure 1 and 2 can be designed in many different ways, depending on the choice of analyzing technology or combination of technologies.

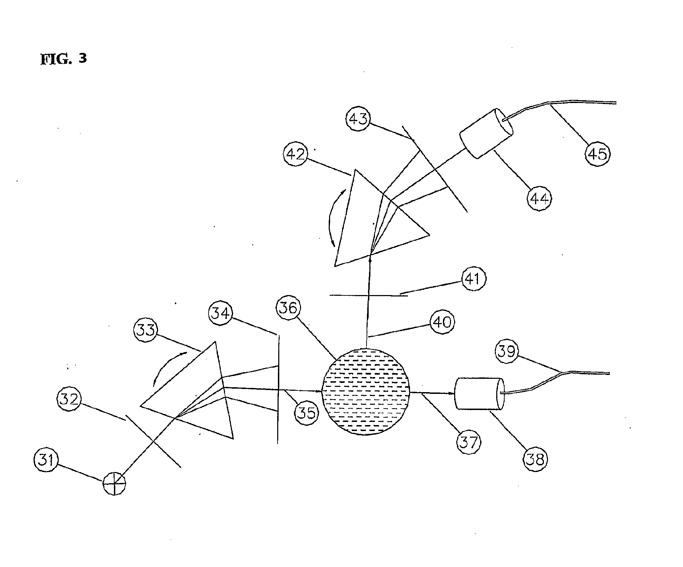

One preferred arrangement for the mentioned optical analyzing techniques is shown in Figure 3. Light is emitted from a polychrome light source (31), collimated by a slit (32) and separated in wavelengths by a prism or grating (33). The prism or grating (33) is rotated to allow only light with one wavelength at a time to go through the second slit (34) and reach the drug solution in the drug analyzing container (36). The monochrome light (35) is passing straight through the drug solution, where the light absorption is different at different wavelengths. The transmitted light (37) reaches a light detector (38) and the measured light intensity signal is fed through a cable (39) to the electronics unit. The prism or grating (33) is rotated to transmit different wavelengths through the slit (34) and the light absorption in the drug solution as a function of the selected wavelength gives the characteristic absorption spectrum, profile, for the drug. The profile for the drug solution is compared to the known profile for the prescribed drug and if they agree, the type of drug has been verified. The magnitude of the absorption at particular wavelengths gives a direct measure of the concentration by use of the Lambert-Beer law: log Iₒ / I = aₛ * b * c, where Iₒ is the incident light intensity, I is the transmitted light intensity, aₛ is the specific absorbance index for the drug at this particular wavelength, b is the path length of the light through the drug solution and c is the concentration of the solution. Since all the parameters are known the concentration is calculated as c = (log Iₒ / I) / aₛ * b.
A second light detection system that analyzes the fluorescent light from the drug solution can also be used, either alone or in combination with the transmitted light detection system. The fluorescent light (40) is collimated by a slit (41) and separated in wavelengths by a prism or grating (42). The prism or grating (42) is rotated to allow only light with one wavelength at a time to go through the second slit (43) and reach a light detector (44). The signal from the light detector (44) is fed through a cable (45) to the electronics unit. The prism or grating (42) is rotated to transmit different wavelengths through the slit (43) and the fluorescent light emission in the drug solution as a function of the selected wavelength gives the characteristic fluorescence spectrum, profile, for the drug. The profile for the drug solution is compared to the known profile for the prescribed drug and if they agree, the type of drug has been verified. This detection arrangement for fluorescence spectra can also be used to detect Raman spectra.

## Claims

1. A method to perform verification of correctness and quality assurance of a drug solution to be administered to a patient by infusion comprising:
(a) providing a drug container (1) containing a sterile drug solution (2),
(b) non-invasively determining in an analysing unit (5) by spectrophotometric methods one or more values of at least one chemical and/or physical property of the drug in the drug solution to generate a profile for the drug,
(c) comparing the generated drug profile with a set of known drug profiles, and (d)if agreement between the generated profile and the profile of a prescribed drug is reached issuing a message that the treatment is safe and infusion of the drug solution can proceed;
**characterized in that**
the drug container (1) is coupled via a first sterile tubing (3) to a sterile analysing container (4; 36), which is coupled to a second tubing (6) connected to a needle (7), and
for the spectrophotometric determination, the analysing container (4; 36) is located in an analysing unit (5), drug solution (2) is flowed through the sterile tubing (3) to the sterile analysing container (4), and the one or more drug property values are determined by absorption spectrophotometry or fluorescence spectrophotometry or a combination thereof, and
if the treatment is safe, infusion of the drug solution is allowed to proceed from the sterile analysing container (4) through the second tubing (6) and the needle (7) into the patient.

2. The method according to claim 1, wherein the spectrophotometric determination is performed by absorption spectrophotometry.

3. The method according to claim 2, wherein the absorption spectrophotometry is performed in a wavelength range selected from ultraviolet, visible and infrared wavelengths.

4. The method according to claim 3, wherein the absorption spectrophotometry is performed in a wavelength range selected from the ultraviolet and visible wavelengths.

5. The method according to claim 3, wherein the absorption spectrophotometry is performed in a wavelength range selected from the ultraviolet wavelengths.

6. The method according to any of the preceding claims, wherein the spectroscopic determination is used for identity measurements of a substance in the drug solution by obtaining a unique profile for each kind of molecule in the drug solution.

7. The method according to any of the preceding claims, wherein the spectroscopic determination is used for comparing the magnitude of the generated profile with the magnitude of known concentration profiles for the substance(s) in the drug solution.

8. The method according to any of the preceding claims, wherein if an agreement is not reached between the obtained profile and the profile of the prescribed drug, a warning message is issued to stop the administration of the drug solution.

9. The method according to any of the preceding claims, wherein the analysing container (4) is coupled to the second tubing (6) via a control valve (14).

10. A device for verification of correctness and quality assurance of a drug solution to be administered to a patient by infusion comprising:
a drug container (1) including the said drug solution (2),
an analysing unit (5) containing optical components (31-34, 38, 41-44) for non-invasive spectroscopic determination of at least one property of a drug in the drug solution (2) and for issuing a signal corresponding to said property and thereby generating a profile for the drug,
a central computation unit (9) to which the signal from the analysing unit (5) is transmitted for comparing the generated drug profile to a set of known drug profiles, and
a presentation unit (8; 10) for displaying a result from comparing the generated profile to the set of known profiles,
**characterized in that**
the drug container (1) is coupled via a first sterile tubing (3) to a sterile analysing container (4), which is coupled to a second tubing (6) and a needle (7) to allow infusion of drug solution (2) from the analysing container (4) into the patient if the treatment is safe,
the analysing unit (5) contains optical components for absorption spectrophotometry or fluorescence spectrophotometry, or a combination thereof, and
the analysing container (4) is located in the analysing unit (5) for spectroscopic determination of said least one property of a drug in drug solution (2) flowed from the drug container (1) to the analysing container (4).

11. The device according to claim 10, wherein an external information network comprising the stored set of known profiles in an external database (11) to which the generated profile is compared, coupled to the central computation unit (9).

12. The device according to claims 10 or 11, wherein the central computation unit (9) is coupled to a patient treatment recording system (12).

13. The device according to any of claims 10 to 12, wherein the central computation unit (9) is coupled to a treatment planning system (13).

14. The device according to any one of claims 10 to 13, wherein the analysing container (4) is coupled to the second tubing (6) via a control valve (14).

## Patentansprüche

1. Verfahren zur Durchführung einer Verifizierung der Korrektheit und Qualitätssicherung einer einem Patienten durch Infusion zu verabreichenden Arzneimittellösung mit:
(a) Bereitstellung eines Arzneimittelbehälters (1), der eine sterile Arzneimittellösung (2) enthält,
(b) nichtinvasive Bestimmung eines oder mehrerer Werte mindestens einer chemischen und/oder physikalischen Eigenschaft des Arzneimittels in der Arzneimittellösung mittels spektrophotometrischer Verfahren in einer Analyseeinheit (5), um ein Profil des Arzneimittels zu erzeugen,
(c) Vergleich des erzeugten Arzneimittelprofils mit mehreren bekannten Arzneimittelprofilen und,
(d) falls eine Übereinstimmung zwischen dem erzeugten Profil und dem Profil eines verordneten Arzneimittels erzielt worden ist, Ausgabe einer Mitteilung, dass die Behandlung sicher ist und eine Infusion der Arzneimittellösung durchgeführt werden kann;
**dadurch gekennzeichnet, dass**
der Arzneimittelbehälter (1) über einen ersten sterilen Schlauch (3) mit einem sterilen Analysebehälter (4;36) verbunden ist, der mit einem zweiten Schlauch (6) verbunden ist, der mit einer Nadel (7) verbunden ist, und
für die spektrophotometrische Bestimmung der Analysebehälter (4;36) in einer Analyseeinheit (5) angeordnet wird, ein Fließen der Arzneimittellösung (2) durch den sterilen Schlauch (3) zum sterilen Analysebehälter (4) ermöglicht wird und der eine oder die mehreren Arzneimitteleigenschaftswerte mittels Absorption-Spektrophotometrie oder Fluoreszenz-Spektrophotometrie oder eine Kombination davon bestimmt werden und,
falls die Behandlung sicher ist, eine Durchführung der Infusion der Arzneimittellösung aus dem sterilen Analysebehälter (4) durch den zweiten Schlauch (6) und die Nadel (7) in den Patienten erlaubt wird.

2. Verfahren nach Anspruch 1, wobei die spektrophotometrische Bestimmung durch Absorptions-Spektrophotometrie erfolgt.

3. Verfahren nach Anspruch 2, wobei die Absorptions-Spektrophotometrie in einem Wellenlängenbereich durchgeführt wird, der aus den ultravioletten, sichtbaren und infraroten Wellenlängen ausgewählt ist.

4. Verfahren nach Anspruch 3, wobei die Absorptions-Spektrophotometrie in einem Wellenlängenbereich durchgeführt wird, der aus den ultravioletten und sichtbaren Wellenlängen ausgewählt ist.

5. Verfahren nach Anspruch 3, wobei die Absorptions-Spektrophotometrie in einem Wellenlängenbereich durchgeführt wird, der aus den ultravioletten Wellenlängen ausgewählt ist.

6. Verfahren nach einem der vorstehenden Ansprüche, wobei die spektrophotometrische Bestimmung verwendet wird, um durch Gewinnen eines eindeutigen Profils für jede Molekülart in der Arzneimittellösung eine Substanz in der Arzneimittellösung zu identifizierten.

7. Verfahren nach einem der vorstehenden Ansprüche, wobei die spektrophotometrische Bestimmung verwendet wird, um für die Substanz(en) in der Arzneimittellösung die Größe des erzeugten Profils mit der Größe bekannter Konzentrationsprofile zu vergleichen.

8. Verfahren nach einem der vorstehenden Ansprüche, wobei in dem Falle, dass keine Übereinstimmung zwischen dem erzeugten Profil und dem Profil des verordneten Arzneimittels erzielt worden ist, eine Warnmitteilung ausgegeben wird, um die Verabreichung der Arzneimittellösung zu beenden.

9. Verfahren nach einem der vorstehenden Ansprüche, wobei der Analysebehälter (4) über ein Steuerventil (14) mit dem zweiten Schlauch (6) verbunden ist.

10. Vorrichtung zur Verifizierung der Korrektheit und Qualitätssicherung einer einem Patienten durch Infusion zu verabreichenden Arzneimittellösung mit:
einem Arzneimittelbehälter (1), der die Arzneimittellösung (2) enthält,
einer optische Komponenten (31-34,38,41-44) aufweisenden Analyseeinheit (5) zur nichtinvasiven spektrophotometrischen Bestimmung mindestens einer Eigenschaft eines Arzneimittels in der Arzneimittellösung (2) und zur Ausgabe eines die Eigenschaft repräsentierenden Signals unter Erzeugung eines Profils des Arzneimittels,
einer zentralen Verarbeitungseinheit (9), zu welcher das Signal von der Analyseeinheit (5) aus gesendet wird, um das erzeugte Arzneimittelprofil mit mehreren bekannten Arzneimittelprofilen zu vergleichen, und
einer Anzeigeeinheit (8;10) zum Anzeigen eines aus dem Vergleich des erzeugten Profils mit den mehreren bekannten Profilen gewonnenen Ergebnisses,
**dadurch gekennzeichnet, dass**
der Arzneimittelbehälter (1) über einen ersten sterilen Schlauch (3) mit einem sterilen Analysebehälter (4) verbunden ist, der mit einem zweiten Schlauch (6) und einer Nadel (7) verbunden ist, um eine Infusion einer Arzneimittellösung (2) aus dem Analysebehälter (4) in den Patienten zu erlauben, wenn die Behandlung sicher ist,
die Analyseeinheit (5) optische Komponenten zur Absorptions-Spektrophotometrie oder Fluoreszenz-Spektrophotometrie oder eine Kombination davon aufweist,
der Analysebehälter (4) in der Analyseeinheit (5) angeordnet wird, um eine spektrophotometrische Bestimmung mindestens einer Eigenschaft eines Arzneimittels in der Arzneimittellösung (2) durchzuführen, die aus dem Arzneimittelbehälter (1) in den Analysebehälter (4) geflossen ist.

11. Vorrichtung nach Anspruch 10, wobei ein externes Datennetz, das die mehreren bekannten Profile, mit welchen das erzeugte Profil verglichen wird, in einer externen Datenbank (11) gespeichert hat, mit der zentralen Verarbeitungseinheit (9) verbunden ist.

12. Vorrichtung nach Anspruch 10 oder 11, wobei die zentrale Verarbeitungseinheit (9) mit einem Patientenbehandlungs-Aufzeichnungssystem (12) verbunden ist.

13. Vorrichtung nach einem der Ansprüche 10 bis 12, wobei die zentrale Verarbeitungseinheit (9) mit einem Behandlungsplansystem (13) verbunden ist.

14. Vorrichtung nach einem der Ansprüche 10 bis 13, wobei der Analysebehälter (4) über ein Steuerventil (14) mit dem zweiten Schlauch (6) verbunden ist.

## Revendications

1. Procédé pour effectuer une vérification de conformité et garantir la qualité d'une solution médicamenteuse à administrer à un patient par perfusion comprenant :
(a) la fourniture d'un contenant de médicament (1) contenant une solution médicamenteuse stérile (2),
(b) la détermination, de manière non invasive dans une unité d'analyse (5) par des procédés spectrophotométriques, d'une ou de plusieurs valeurs d'au moins une propriété chimique et/ou physique du médicament dans la solution médicamenteuse pour générer un profil pour le médicament,
(c) la comparaison du profil de médicament généré avec un ensemble de profils de médicament connus, et
(d) si une concordance entre le profil généré et le profil d'un médicament prescrit est obtenue, l'émission d'un message indiquant que le traitement est sûr et que la perfusion de la solution médicamenteuse peut avoir lieu ;
**caractérisé en ce que**
le contenant de médicament (1) est couplé par l'intermédiaire d'une première tubulure stérile (3) à un contenant d'analyse stérile (4 ; 36), qui est couplé à une deuxième tubulure (6) reliée à une aiguille (7), et
pour la détermination spectrophotométrique, le contenant d'analyse (4 ; 36) est placé dans une unité d'analyse (5), la solution médicamenteuse (2) est mise en circulation à travers la tubulure stérile (3) vers le contenant d'analyse stérile (4), et les une ou plusieurs valeurs de propriété de médicament sont déterminées par spectrophotométrie à absorption ou par spectrophotométrie de fluorescence ou par une combinaison de celles-ci, et
si le traitement est sûr, la perfusion de la solution médicamenteuse peut avoir lieu du contenant d'analyse stérile (4) à travers la deuxième tubulure (6) et l'aiguille (7) dans le patient.

2. Procédé selon la revendication 1, dans lequel la détermination spectrophotométrique est effectuée par spectrophotométrie à absorption.

3. Procédé selon la revendication 2, dans lequel la spectrophotométrie à absorption est effectuée dans une plage de longueurs d'ondes sélectionnée parmi les longueurs d'ondes ultraviolettes, visibles et infrarouges.

4. Procédé selon la revendication 3, dans lequel la spectrophotométrie à absorption est effectuée dans une plage de longueurs d'ondes sélectionnée parmi les longueurs d'ondes ultraviolettes et visibles.

5. Procédé selon la revendication 3, dans lequel la spectrophotométrie à absorption est effectuée dans une plage de longueurs d'ondes sélectionnée parmi les longueurs d'ondes ultraviolettes.

6. Procédé selon l'une quelconque des revendications précédentes, dans lequel la détermination spectroscopique est utilisée pour des mesures d'identité d'une substance dans la solution médicamenteuse en obtenant un profil unique pour chaque type de molécule dans la solution médicamenteuse.

7. Procédé selon l'une quelconque des revendications précédentes, dans lequel la détermination spectroscopique est utilisée pour comparer l'amplitude du profil généré avec l'amplitude de profils de concentration connus pour la ou les substances dans la solution médicamenteuse.

8. Procédé selon l'une quelconque des revendications précédentes, dans lequel, si une concordance n'est pas obtenue entre le profil obtenu et le profil du médicament prescrit, un message d'avertissement est émis pour arrêter l'administration de la solution médicamenteuse.

9. Procédé selon l'une quelconque des revendications précédentes, dans lequel le contenant d'analyse (4) est couplé à la deuxième tubulure (6) par l'intermédiaire d'une vanne de régulation (14).

10. Dispositif pour une vérification de conformité et une garantie de qualité d'une solution médicamenteuse à administrer à un patient par perfusion comprenant :
un contenant de médicament (1) comprenant ladite solution médicamenteuse (2),
une unité d'analyse (5) contenant des composants optiques (31 à 34, 38, 41 à 44) pour la détermination spectroscopique non invasive d'au moins une propriété d'un médicament dans la solution médicamenteuse (2) et pour émettre un signal correspondant à ladite propriété et de ce fait générer un profil pour le médicament,
une unité centrale de calcul (9) à laquelle le signal provenant de l'unité d'analyse (5) est transmis pour comparer le profil de médicament généré à un ensemble de profils de médicament connus, et
une unité de présentation (8 ; 10) pour afficher un résultat de la comparaison du profil généré avec l'ensemble de profils connus,
**caractérisé en ce que**
le contenant de médicament (1) est couplé par l'intermédiaire d'une première tubulure stérile (3) à un contenant d'analyse stérile (4), qui est couplé à une deuxième tubulure (6) et à une aiguille (7) pour permettre la perfusion de la solution médicamenteuse (2) du contenant d'analyse (4) dans le patient, si le traitement est sûr,
l'unité d'analyse (5) contient des composants optiques pour une spectrophotométrie à absorption ou une spectrophotométrie de fluorescence ou une combinaison de celles-ci, et
le contenant d'analyse (4) est situé dans l'unité d'analyse (5) pour la détermination spectroscopique de ladite au moins une propriété d'un médicament dans la solution médicamenteuse (2) mise en circulation du contenant de médicament (1) vers le contenant d'analyse (4).

11. Dispositif selon la revendication 10, dans lequel un réseau d'informations externe, comprenant l'ensemble de profils connus mémorisé dans une base de données externe (11) auxquels le profil généré est comparé, est couplé à l'unité centrale de calcul (9).

12. Dispositif selon la revendication 10 ou 11, dans lequel l'unité centrale de calcul (9) est couplée à un système d'enregistrement de traitement de patient (12).

13. Dispositif selon l'une quelconque des revendications 10 à 12, dans lequel l'unité centrale de calcul (9) est couplée à un système de planification de traitement (13).

14. Dispositif selon l'une quelconque des revendications 10 à 13, dans lequel le contenant d'analyse (4) est couplé à la deuxième tubulure (6) par l'intermédiaire d'une vanne de régulation (14).
